(19) Europäisches Patentamt
European Patent Office
Office européen des brevets

(11) Publication number: **0 177 457 B1**

# (12) EUROPEAN PATENT SPECIFICATION

(45) Date of publication of the patent specification: 27.12.89

(51) Int. Cl.⁴: A01N 47/36, C07D 271/08

(21) Application number: 85830214.4

(22) Date of filing: 09.08.85

(54) **Compositons containing heterocyclic compounds and their use as herbicides.**

(30) Priority: 29.08.84 IT 2245084

(43) Date of publication of application: 09.04.86 Bulletin 86/15

(45) Publication of the grant of the patent: 27.12.89 Bulletin 89/52

(84) Designated Contracting States: AT BE CH DE FR GB LI LU NL SE

(56) References cited:
EP-A- 0 132 680
DE-A- 2 436 179

(73) Proprietor: ENICHEM SYNTHESIS S.p.A., Via Ruggero Settimo 55, I-90139 Palermo(IT)

(72) Inventor: Calvino, Rosella, Via Petrarca 29, I-10100 Torino(IT)
Inventor: Fruttero, Roberta, Via Cernaia 8, I-12038 Savigliano (Cuneo)(IT)
Inventor: Messori, Vittorio, Piazza Chiaves 10, I-10100 Torino(IT)
Inventor: Rodio, Francesco, Via Pagliani 1, I-10100 Torino(IT)

(74) Representative: Rambelli, Paolo et al, Jacobacci-Casetta & Perani S.p.A. Via Alfieri 17, I-10121 Torino(IT)

EP 0 177 457 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to composition containing heterocyclic compounds having a urea group and having herbicidal activities, to their use in the control and destruction of infestant plants and to the process for preparing them.

It is known that there is a great need in agriculture for new, alternative herbicidal products which have high activities in the elimination of weeds together with a substantial lack to toxicity to man and animals.

According to the present invention these requirements are satisfied by means of new herbicidal compounds having a heterocyclic structure and having a urea group attached to the heterocyclic structure.

Accordingly the present invention provides a method for controlling the growth of infestant plants in pre-emergence or post-emergence, characterised in that the infested ground is treated with an effective amount of herbicidal compound or of a composition containing a herbicidal compound, selected from those which can be defined by the general formula

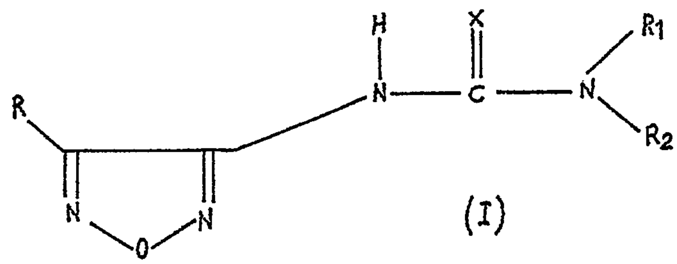

(I)

in which:
X is oxygen or sulphur;
R is a linear or branched alkyl group containing from 1 to 6 carbon atoms;
– the phenyl group;
– a phenyl group having a substituent, or several substituents which may be identical or different from each other, selected from the halogens and alkyl ($C_1$–$C_4$), oxyalkyl ($C_1$–$C_4$), halogenoalkyl ($C_1$–$C_4$) and nitro groups;
$R_1$ is a linear or branched alkyl group containing from 1 to 10 carbon atoms;
– a cycloalkyl group containing from 3 to 8 carbon atoms;
– the phenyl group;
– the benzyl group;
– the naphthyl group; or
– a phenyl, benzyl or naphthyl group having one or more substituents, which may be identical or different from each other, selected from halogen atoms and ($C_1$–$C_4$) alkyl, ($C_1$–$C_4$) oxyalkyl and, ($C_1$–$C_4$) halogenoalkyl and nitro group;
$R_2$ is hydrogen;
– a linear or branched alkyl group containing from 1 to 6 carbon atoms, or
– the phenyl group;
or $R_1$ and $R_2$, taken together with the nitrogen atom to which they are connected, form an 8-membered heterocyclic ring.

The invention further provides novel herbicidal compositions comprising one or more adjuvants including diluents, wetting agents and/or solvents with from 5 to 95% by weight of a herbicidal compound having the general formula (I) as above defined wherein R is a linear or branched alkyl group containing from 1 to 6 carbon atoms and wherein X, $R_1$ and $R_2$ are as above defined.

In the definitions above, halogen is intended to mean fluorine, chlorine, bromine or iodine.

Examples of substituents in the general formula (I) given above are:
R = methyl
$R_1$ = methyl, n-propyl, isopropyl, cyclohexyl, phenyl, 3-trifluoromethylphenyl, 4-fluorophenyl and naphthyl
$R_2$ = hydrogen and methyl.

Preferred compounds according to the present invention are those belonging to the general formula (I) above, in which:
X is oxygen
R is methyl
$R_1$ is linear or branched $C_1$–$C_5$ alkyl, cyclohexyl or phenyl;
$R_2$ is hydrogen.

Specific examples of compounds which fall within the general formula (I) above are:
N-(4-methylfurazan-3-yl)-N'-methylurea;
N-(4-methylfurazan-3-yl)-N'-n-propylurea;
N-(4-methylfurazan-3-yl)-N'-isopropylurea;

N-(4-methylfurazan-3-yl)-N'-tert-butylurea;
N-(4-methylfurazan-3-yl)-N,N'-dimethylurea;
N-(4-methylfurazan-3-yl)-N'-cyclohexylurea;
N-(4-methylfurazan-3-yl)-N'-phenylurea;
N-(4-methylfurazan-3-yl)-N'-(3-trifluoromethylphenyl)urea;
N-(4-methylfurazan-3-yl)-N'-(4-fluorophenyl)urea;
N-(4-methylfurazan-3-yl)-N'-naphthylurea;
N-(4-methylfurazan-3-yl)-N'-phenylthiourea.

Same of the compounds represented by the general formula (I) such as
Urea-N-phenyl-N'-(phenylfurazanyl) Registry Number 49 615-84-1;
Urea-N-phenyl-N'-(methyl-furazanyl) Registry Number 63 558-48-5;
Thiourea-N-phenyl-N'-(methyl-furazanyl) Registry Number 64 821-98-3;
are known from the literature which describes their synthesis, their physical and chemical characteristics and, in some cases, their pharmacological properties. In this respect one is referred to the following literature:

- G. Cusmano and T. Tiberio, Gazz. Chim. Ital. 81, 106 (1951);
- M. Ruccia, N. Vivona and G. Cusmano, J.C.S. Perkin I (1977), 589;
- M. Ruccia, N. Vivona and G. Cusmano, J.C.S. Perkin I (1977), 1616;
- S. African Patent No. 680.779.

The present invention is based on the discovery that these known compounds and other new compounds falling within the general formula (I) have herbicidal activities with a wide spectrum of action against infestant plants, but are substantially innocuous to man and animals. The compounds (I) may be synthesised by the following reaction scheme.

(a) 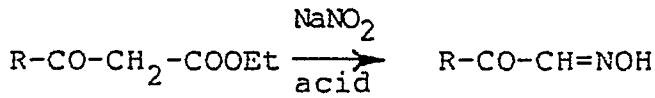


or:

(a') 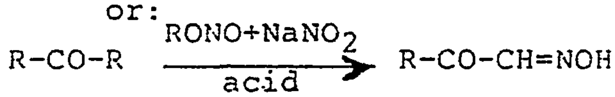


(b) 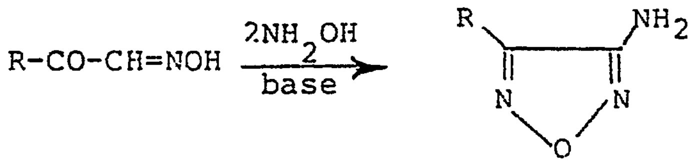


(c) 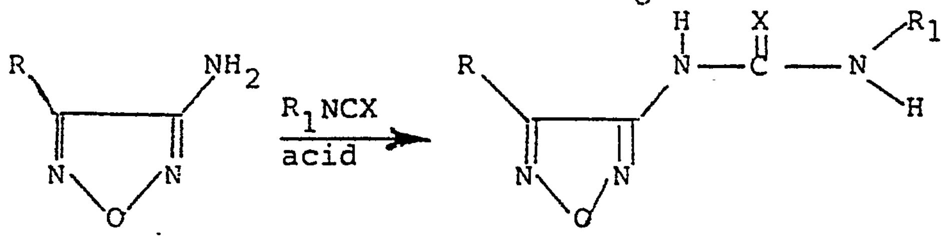


or:

(c') 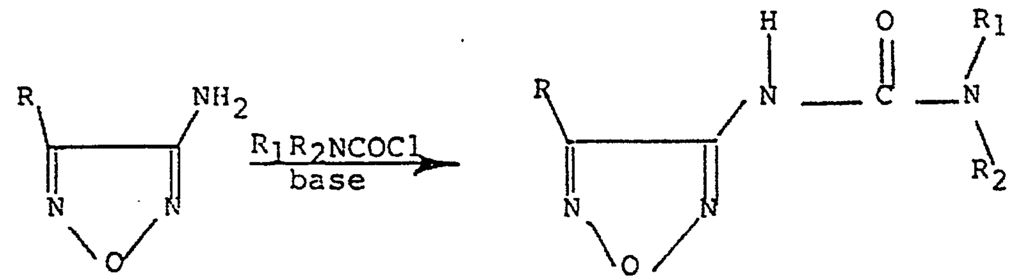


or:

(c'') 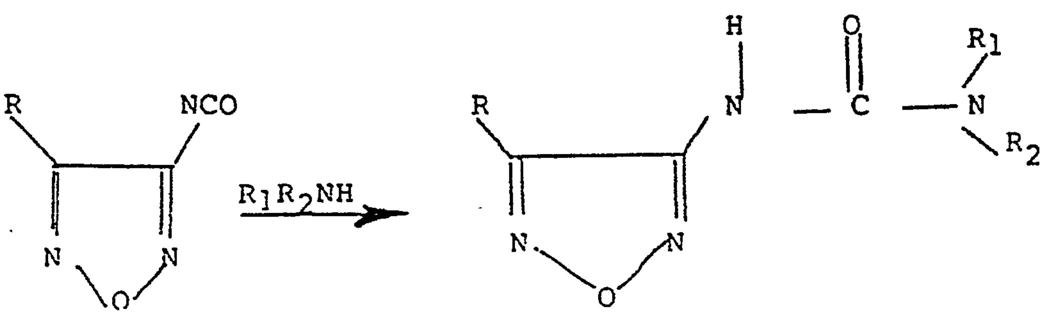

In the formulae above, X, R, $R_1$ and $R_2$ have the meaning indicated previously.

Typically the basic step of the process of the present invention for preparing the compounds having the general formula (I) is consisting of reacting a 3-amine- (4-R-substituted)-furazan (II) with an isocyanate $R_1$-NCO (III),wherein R and $R_1$ have the same meaning of the general formula (I), with a (III) to (II) molar ratio from 1:1 to 1.2:1, at a temperature of from 0° to 100° C, for a time of from 1 to 24 hours and of recovering the compound (I) from the reaction mixture by filtration.

The reaction can be run in an inert organic solvent in which case the preferred solvents are dichloroethane, diethylether or dimethylformamide.

The compounds(I) according to the present invention heve good herbicidal activities when used both under pre-emergence and post-emergence conditions in doses of from 0.1 kg/hectare up to a maximum of 5kg/hectare. Moreover, the herbicidal activity of the compounds (I) is highly selective with regard to crops belonging to the grass family (gramineae), for example towards wheat and maize, up to doses of 3 kg/hectare; at higher doses the herbicidal activity of the compounds tends to become total.

The compounds (I) of the present invention are herbicides which interfere both with the seed germination phase and with the subsequent development of the embryo plants. They have good herbicidal activities and may thus be used conveniently both in pre-emergence and post-emergence weed-killing in

crops, in accordance with the different degrees of selectivity shown and the biological cycles of the infestants.

The compounds (I) of the present invention may be applied by the usual methods, in solution, suspension or emulsion, as powders or as granules, according to the chosen application, as long as the active principle is finely divided.

The compositions of the present invention are typically prepared by the mixture of the active ingredient with an adjuvant including diluents, fillers, extenders and conditioning agents to provide the compositions in the form of finely - divided solid particles, granules, solutions, dispersions or emulsions. The active ingredient may thus be used with an adjuvant such as a finely-divided solid, an organic liquid, water, a wetting agent, a dispersing agent or any suitable combination thereof. The herbicidal compositions of the present invention, particularly liquids or soluble powders, preferably contain one or more surface active agents as conditioning agents in quantities sufficient to render a particular composition readily dispersible in water or oil. The incorporation of a surface active agent in the composition greatly improves its effectiveness. By "surface active agents" are meant wetting agents, dispersing agents, suspending agents, and emulsifying agents. Anionic, cationic or non-ionic agents may be used equally well.

Preferred wetting agents are alkylbenzene sulphonates, alkylnaphthalene sulphonates, aliphatic alcohol sulphonates, polyoxyethylene derivatives of alkylphenols (particularly isooctylphenol and nonylphenol. Compositions of powders dispersible in water may be made with one or more active ingredients, an inert solid filler and one or more wetting and dispersing agents. The solid inert fillers are usually of mineral origin, for example natural clays, diatomaceous earths and synthetic minerals derived from silica. Examples of such fillers comprise kaolin, attapulgite and synthetic magnesium silicate. The powders of the present invention which are dispersible in water usually contain about 5 to about 95 parts by weight of the active ingredient, from about 0.25 to about 25 parts by weight of the wetting agent, from about 0.25 to about 25 parts by weight of the dispersing agent and from about 4.5 to about 94.5 parts by weight of the solid inert extender, all the parts being with reference to the total weight of the composition.

The aqueous suspensions may be prepared by mixing and grinding together an aqueous suspension of the active ingredient which is insoluble in water to obtain a concentrated suspension of very finely divided particles. The resulting concentrated aqueous suspension has extremely small particles such that when it is diluted and sprayed the coating is very uniform.

The emulsifiable oils are generally solutions of the active ingredient in solvents which are immiscible or slightly miscible with water, together with a surfactant active agent. Solvents suitable for the active ingredients of the present invention include hydrocarbons and ethers, esters and ketones which are immiscible with water. The composition of the emulsifiable oil generally contains from about 5 to about 95 parts by weight of the active ingredient, from about 1 to about 50 parts by weight of surfactant and from about 4 to about 94 parts by weight of solvent, all the parts being based on the total weight of the emulsifiable oil.

The experimental examples which follow are given by way of example.

Example 1

Synthesis of N-(4-methylfurazan-3-yl)-N'-phenylurea

4-methyl-3-aminofurazan is first prepared from isonitrosoacetone by the general method described in Berichte 13, 1328 (1882), followed by cyclization as described in Gazz.Chim.Ital.81,106 (1951).

10g (about 0.1 moles) of 4-methyl-3-aminofurazan are introduced into a reactor fitted with an agitator, reflux condenser, loading phial and dry ice trap and 12 ml of recently-distilled phenylisocyanate are added through the loading phial. The mixture is heated over an oil bath to a temperature of 100°C for about one hour. The reaction mass, after cooling, is taken up in anhydrous toluene and subsequently filtered and the separated solid is dried. The crude product obtained is crystallized from ethanol and 17.5g of white crystals of the title compound are obtained with a melting point of 215°C.

Example 2

Preparation of N-(4-methylfurazan-3-yl)-N'-isopropylurea

5g (about 0.05 moles)of -4-methyl-3-aminofurazan, 40 ml of anhydrous sym-dichloroethane and, through a loading phial, 7.5 ml of isopropylisocyanate are introducted into a reactor fitted with an agitator and a reflux condenser. The mass is heated under reflux for 24 hours, the reaction being catalysed by traces of anhydrous trifluoroacetic acid. The solvent is then evaporated and the precipitate obtained is washed repeatedly with hot water. The title compound is thus obtained (85% yield with respect to the theoretical value) and, crystallized from ethanol, produces white crystals with a melting point of 199-200°C.

Examples 3-6

These are carried out similarly to Example 2 (general reaction scheme (c)), 4-methyl-3-aminofurazan being reacted with suitable isocyanates, and the following compounds are obtained:
Ex. 3) N-(4-methylfurazan-3-yl)-N'-methylurea; m.p. 217-218°C (crystallized from ethanol-$H_2O$)
Ex. 4) N-(4-methylfurazan-3-yl)-N'-n-propylurea; m.p. 167-168°C (crystallized from ethanol-$H_2O$)
Ex. 5) N-(4-methylfurazan-3-yl)-N'-tert-butylurea; m.p. 161-162°C (crystallized from ethanol-$H_2O$)
Ex. 6) N-(4-methylfurazan-3-yl)-N'-cyclohexylurea; m.p. 200-201°C (crystallized from ethanol-$H_2O$)

Examples 7-9

These are carried out similarly to Example 1, the following compounds being obtained:
Ex. 7) N-(4-methylfurazan-3-yl)-N'-(3-trifluoromethylphenyl) urea; m.p. 206-207°C (crystallized from ethanol)
Ex.8) N-(4-methylfurazan-3-yl)-N'-(4-fluorophenyl)urea; m.p. 214-215°C (crystallized from ethanol)
Ex. 9) N-(4-methylfurazan-3-yl)-N'-naphthylurea; m.p. 219-220°C (crystallized from ethanol)

Example 10

Preparation of N-(4-methylfurazan-3-yl)-N'-phenylthiourea

2.5 g (about 0.025 moles) of 4-methyl-3-aminofurazan, 6 ml di dimethylformamide, 3.4 g of phenylisothiocyanate and traces of hydrochloric acid as a catalyst are loaded into a glass flask fitted with a sealed stopper. The flask is closed and placed in an oven at 30°C for 40 hours. The reaction mixture is poured into cold water under agitation. The precipitate is filtered and dissolved in a little benzene. The benzene solution is filtered and petroleum ether is added to the filtrate causing the precipitation of a solid which is crystallized from ethanol, giving 2 g of crystals of the title product, with a melting point of 124-125°C (crystallized from ethanol).

Example 11

The compound N-(4-methylfurazan-3-yl)-N',N'-dimethylurea is prepared by the general reaction scheme (c'), and has a melting point of 106°C (crystallized from cyclohexane).
The structure of the compounds of Examples 1 to 11 is confirmed by routine spectroscopy (IR, NMR), mass spectrometry and elementary analysis (values within the range ± 3% of the theoretical).

Evaluation of the herbicidal activity

All the plants were cultivated in greenhouses in sterilized, fertilized ground at a controlled temperature (20±5°C) and with a relative humidity of 60±10%. All the species were also cultivated in plastic containers having a size of 22 x 15 x 6 cm. The compounds described in the above examples were formulated as wettable powders, suspension or water-acetone solutions with 10% of the active principle.

Pre-emergence test

The containers with a suitable number of seeds of each species were treated 24 hours after sowing.

Post-emergence test

The plants were treated when they had reached the stage of 2-3 leaves, about 14 days after sowing.
The treatment was carried out both pre-emergence and post-emergence with 2.5 and 5kg/hectare doses, with the use of an Oxford precision pump at pressures of 5 psi (0.35 bar).
The evaluation was carried out ten days after treatment for the post-emergence test and 15-20 days after treatment for the pre-emergence test.
The effect of the active principle was evaluated according to a scale of from 0 to 4 in which:

0 = no damage;
1 = 25% damage;
2 = 50% damage;
3 = 75% damage;
4 = 100% damage.

The results are given in Table 1 below and it may be seen that the herbicidal compounds of the present invention are, in general, active against vegetable species belonging to the dicotyledon family, while having good selectivity towards the grass family (gramineae), that is at lower doses.

At doses above about 3 kg/hectare there is a general tendency towards a reduction in the selectivity towards grasses.

TABLE 1

| SPECIES | TREATMENT | Ex. 1 | Ex. 2 | Ex. 3 | Ex. 4 | Ex. 5 | Ex. 6 | Ex. 7 | Ex. 8 | Ex. 9 | Ex. 10 | Ex. 11 |
|---|---|---|---|---|---|---|---|---|---|---|---|---|
| Beta V. | pre-emergence | 2 | 3 | 2 | 2 | 2 | 1 | 1 | 2 | 0 | 0 | 0 |
| | post-emergence | 4 | 3 | 3 | 4 | 4 | 4 | 4 | 3 | 4 | 4 | 1 |
| Pisum S. | pre-emergence | 1 | 4 | 0 | 0 | 1 | 1 | 0 | 0 | 0 | 0 | 0 |
| | post-emergence | 4 | 3 | 3 | 3 | 3 | 4 | 3 | 3 | 4 | 4 | 0 |
| Solanum L. | pre-emergence | 4 | 4 | 4 | 4 | 2 | 2 | 1 | 4 | 0 | 0 | 1 |
| | post-emergence | 4 | 4 | 4 | 4 | 4 | 4 | 4 | 4 | 4 | 4 | 2 |
| Cichorium I | pre-emergence | 4 | 4 | 2 | 4 | 2 | 3 | 1 | 2 | 0 | 0 | 0 |
| | post-emergence | 4 | 4 | 2 | 4 | 4 | 3 | 3 | 2 | 1 | 4 | 2 |
| Zea Mais | pre-emergence | 2 | 0 | 0 | 2 | 0 | 0 | 0 | 0 | 0 | 0 | 0 |
| | post-emergence | 3 | 1 | 0 | 2 | 1 | 0 | 0 | 0 | 1 | 1 | 0 |
| Linum U. | pre-emergence | 2 | 2 | 3 | 4 | 2 | 2 | 1 | 3 | 0 | 0 | 0 |
| | post-emergence | 4 | 1 | 4 | 4 | 4 | 4 | 4 | 4 | 2 | 4 | 0 |
| Lolium I. | pre-emergence | 1 | 1 | 0 | 2 | 1 | 0 | 0 | 0 | 0 | 0 | 0 |
| | post-emergence | 2 | 1 | 1 | 2 | 2 | 1 | 1 | 0 | 0 | 1 | 1 |
| Avena S. | pre-emergence | 2 | 1 | 0 | 2 | 1 | 0 | 0 | 0 | 0 | 0 | 0 |
| | post-emergence | 2 | 1 | 1 | 2 | 2 | 1 | 1 | 0 | 0 | 1 | 1 |
| | DOSE (kg/ha) | 5 | 2.5 | 5 | 5 | 2.5 | 2.5 | 5 | 5 | 5 | 5 | 5 |

Examples of the preparation of compositions containing the herbicidal compounds of the present invention are given below.

Preparation of concentrates that can be emulsified

Ten parts by weight of active principle are dissolved in 80 parts by weight of a mixture of cyclohexanone and xylene (50/50 by volume) under slow, continuous agitation, at a temperature of from ambient (20-25°C) to about 50°C. The mixture is left under agitation until the whole of the solid has dissolved. The solution obtained is filtered through a sintered silica plate filter to remove any insoluble residue and then ten parts by weight of emulsifying agent constituted by a mixture (1:1 by weight) of ionic surfactant and non-ionic surfactant is added at ambient temperature with agitation. The ionic surfactant is constituted by the calcium salt of dodecylbenzene sulphonic acid. The non-ionic surfactant is constituted by ether/ester of palmitic or oleic acid, condensed with 8-15 moles of ethylene oxide.

Preparation of wettable powders

To ten parts of weight of ground active principle are added 80 parts by weight of a mixture of inert substances constituted by 60 parts by weight of kaolin, 15 parts by weight of diatomaceous earth and 5 parts by weight of colloidal silica. The mixture obtained is thoroughly homogenised in a ball mill, agitation being maintained for 2 hours. The product obtained is ground to a grain size of less than 40 microns by grinding in a blade mill, a composition thus being obtained which is suitable for dispersion in water.

Preparation of concentrated suspensions

The active principle is dispersed in a base liquid containing a surfactant. More particularly, 40 parts by weight of active principle in the form of a very fine powder, such as that obtained by grinding in a blade mill or micronization in an air jet mill are mixed slowly in a homogenizer with the base liquid constituted by 45 parts by weight of demineralised water, 5 parts by weight of ethylene glycol and 5 parts by weight of surfactant. The latter is a calcium salt of dodecylbenzene sulphonic acid mixed with polyoxyethylene monosterarate.

A paste is thus obtained which may be further refined by passage through a ball mill.

## Claims

1. Herbicidal composition containing one or more adjuvants including diluents, wetting agents, solvents, with from 5 to 95% by weight of a herbicidal compound selected from those definable by the following general formula:

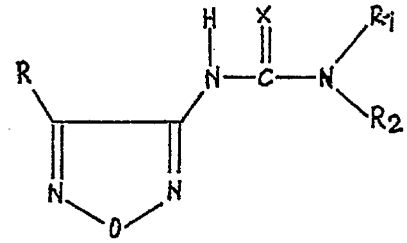

in which:
X is oxygen or sulphur;
R is a linear or branched alkyl group containing from 1 to 6 carbon atoms;
$R_1$ is a linear or branched alkyl group containing from 1 to 10 carbon atoms;
– a cycloalkyl group containing from 3 to 8 carbon atoms;
– the phenyl group;
– the naphthyl group; or
– a phenyl, benzyl or naphthyl group having one or more substituents, which may be identical or different from each other, selected from halogen atoms and ($C_1$–$C_4$) alkyl, ($C_1$–$C_4$) oxyalkyl and ($C_1$–$C_4$) halogenoalkyl and nitro groups;
$R_2$ is hydrogen;
– a linear or branched alkyl group containing from 1 to 6 carbon atoms, or
– the phenyl group;
or $R_1$ and $R_2$, taken together with the nitrogen atom to which they are connected, form an 8-membered heterocyclic ring.

2. Composition according to Claim 1, characterised in that R in the general formula is methyl.

3. Composition according to Claim 1, characterised in that the X in the general formula is oxygen.

4. Composition according to Claim 1, characterised in that the $R_1$ in the general formula is a linear or branched $C_1$–$C_5$ alkyl group; the cyclohexyl or the phenyl group.

5. Composition according to Claim 1, characterised in that the $R_2$ in the general formula is hydrogen.

6. Method for controlling the growth of infestant plants, in pre-emergence or post-emergence, characterised in that the infested ground is treated with an effective amount of a herbicidal compound or of a composition containing a herbicidal compound, selected from those having the general formula:

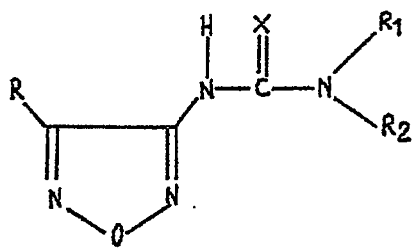

in which:
X is oxygen or sulphur;
R is a linear or branched alkyl group containing from 1 to 6 carbon atoms;
– the phenyl group;
– a phenyl group having a substituent, or several substituents which may be identical or different from each other, selected from the halogens and alkyl ($C_1$–$C_4$), oxyalkyl ($C_1$–$C_4$), halogenoalkyl ($C_1$–$C_4$) and nitro groups;
$R_1$ is a linear or branched alkyl group containing from 1 to 10 carbon atoms;
– a cycloalkyl group containing from 3 to 8 carbon atoms;
– the phenyl group;
– the benzyl group;
– the naphthyl group; or
– a phenyl, benzyl or naphthyl group having one or more substituents, which may be identical or different from each other, selected from halogen atoms and ($C_1$–$C_4$) alkyl, ($C_1$–$C_4$) oxyalkyl and ($C_1$–$C_4$) halogenoalkyl and nitro groups;
$R_2$ is hydrogen;

– a linear or branched alkyl group containing from 1 to 6 carbon atoms, or
– the phenyl group;
or $R_1$ and $R_2$, taken together with the nitrogen atom to which they are connected, form an 8-membered heterocyclic ring.

7. Method according to Claim 6, characterised in that the ground is treated with from 0.1 to 5 kg/hectare of the herbicidal compound.

**Patentansprüche**

1. Herbizide Zusammensetzung, die ein oder mehrere Hilfsmittel, wie Verdünnungs-, Netz-, Lösungsmittel, und 5 bis 95 Gew-% einer herbiziden Verbindung enthält, die aus jenen Verbindungen ausgewählt ist, die der folgenden allgemeinen Formel entsprechen

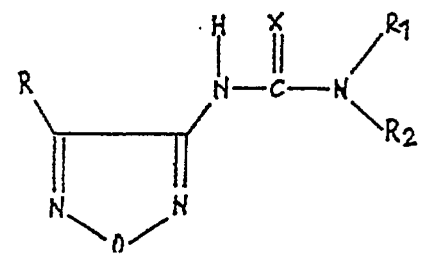

in welcher X für Sauerstoff oder Schwefel,
R für eine lineare oder verzweigte Alkylgruppe mit 1 bis 6 Kohlenstoffatomen,
$R_1$ für eine lineare oder verzweigte Alkylgruppe mit 1 bis 10 Kohlenstoffatomen, eine Zykloalkylgruppe mit 3 bis 8 Kohlenstoffatomen, die Phenylgruppe, die Benzylgruppe, die Naphthylgruppe oder eine Phenyl-, Benzyl- oder Naphthylgruppe mit einem oder mehreren Substituenten, die ident oder voneinander verschieden sein können und ausgewählt sind aus den Halogenatomen, den $(C_1–C_4)$Alkyl-, $(C_1–C_4)$Oxyalkyl- und $(C_1–C_4)$Halogenalkylgruppen sowie der Nitrogruppe,
$R_2$ für Wasserstoff, eine lineare oder verzweigte Alkylgruppe mit 1 bis 6 Kohlenstoffatomen oder die Phenylgruppe steht oder
$R_1$ und $R_2$ gemeinsam mit dem Stickstoffatom, mit dem sie verbunden sind, einen 8-gliedrigen heterozyklischen Ring bilden.

2. Zusammensetzung nach Anspruch 1, dadurch gekennzeichnet, daß R in der allgemeinen Formel für Methyl steht.

3. Zusammensetzung nach Anspruch 1, dadurch gekennzeichnet, daß X in der allgemeinen Formel für Sauerstoff steht.

4. Zusammensetzung nach Anspruch 1, dadurch gekennzeichnet, daß $R_1$ in der allgemeinen Formel eine lineare oder verzweigte $(C_1–C_5)$Alkylgruppe, die Zykloalkylgruppe oder die Phenylgruppe ist.

5. Zusammensetzung nach Anspruch 1, dadurch gekennzeichnet, daß $R_2$ in der allgemeinen Formel für Wasserstoff steht.

6. Verfahren zur Bekämpfung des Wachstums von unerwünschten Pflanzen vor dem Aufgehen oder nach dem Aufgehen, dadurch gekennzeichnet, daß der befallene Boden mit einer wirksamen Menge einer herbiziden Verbindung oder einer eine herbizide Verbindung enthaltenden Zusammensetzung behandelt wird, wobei die herbizide Verbindung ausgewählt ist aus jenen der allgemeinen Formel

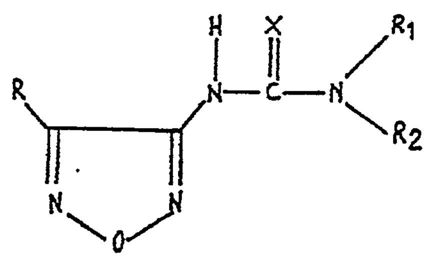

worin X für Sauerstoff oder Schwefel,
R für eine lineare oder verzweigte Alkylgruppe mit 1 bis 6 Kohlenstoffatomen, die Phenylgruppe, eine Phenylgruppe mit einem oder mehreren identen oder voneinander verschiedenen Substituenten, die ausgewählt sind aus den Halogenen, $(C_1–C_4)$Alkyl-, $(C_1–C_4)$Oxyalkyl-, $(C_1–C_4)$Halogenalkylgruppen und der Nitrogruppe, und
$R_1$ für eine lineare oder verzweigte Alkylgruppe mit 1 bis 10 Kohlenstoffatomen, eine Zykloalkylgruppe mit 3 bis 8 Kohlenstoffatomen, die Phenylgruppe, die Benzylgruppe, die Naphthylgruppe oder eine Phenyl-,

Benzyl- oder Naphthylgruppe mit einem oder mehreren Substituenten, die ident oder voneinander verschieden sein können und ausgewählt sind aus den Halogenatomen, den ($C_1$–$C_4$)Alkyl-, ($C_1$–$C_4$)Oxyalkyl- und ($C_1$–$C_4$)Halogenalkylgruppen sowie der Nitrogruppe, und

$R_2$ für Wasserstoff, eine lineare oder verzweigte Alkylgruppe mit 1 bis 6 Kohlenstoffatomen oder die Phenylgruppe steht oder

$R_1$ und $R_2$ gemeinsam mit dem Stickstoffatom, mit dem sie verbunden sind, einen 8-gliedrigen heterozyklischen Ring bilden.

7. Verfahren nach Anspruch 6, dadurch gekennzeichnet, daß der Boden mit 0,1 bis 5 kg/ha der herbiziden Verbindung behandelt wird.

## Revendications

1. Composition d'herbicides contenant un ou plusieurs adjuvants, y compris diluants, agents mouillants, solvants, avec de 5 à 95% en poids d'un composé herbicide choisi parmi ceux définissables par la formule générale suivante:

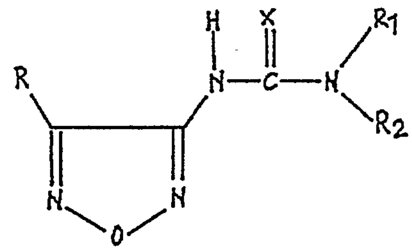

dans laquelle:

X est un atome d'oxygène ou de soufre;

R est un groupe alkyle linéaire ou ramifié, comportant de 1 à 6 atomes de carbone;

$R_1$ est un groupe alkyle linéaire ou ramifié, comportant de 1 à 10 atomes de carbone;

un groupe cycloalkyle comportant de 3 à 8 atomes de carbone;

le groupe phényle;

le groupe benzyle;

le groupe naphthyle; ou bien

un groupe phényle, benzyle ou naphthyle, présentant un ou plusieurs substituants qui peuvent être identiques ou différents les uns des autres, choisis parmi les atomes d'halogène et les groupes alkyle en alcoxy en $C_{1-4}$, halogénoalkyle en $C_{1-4}$ et nitro;

$R_2$ est un atome d'hydrogène;

un groupe alkyle linéaire ou ramifié comportant de 1 à 6 atomes de carbone, ou

le groupe phényle; ou bien $R_1$ et $R_2$, pris conjointement avec l'atome d'azote auquel ils sont liés, forment un noyau hétérocyclique à 8 chaînons.

2. Composition conforme à la revendication 1, caractérisée en ce que R, dans la formule générale, est un groupe méthyle.

3. Composition conforme à la revendication 1, caractérisée en ce que X, dans la formule générale, est un atome d'oxygène.

4. Composition conforme à la revendication 1, caractérisée en ce que $R_1$, dans la formule générale, est un groupe alkyle en $C_{1-5}$ linéaire ou ramifié, le groupe cyclohexyle ou le groupe phényle.

5. Composition conforme à la revendication 1, caractérisée en ce que $R_2$, dans la formule générale, est un atome d'hydrogène.

6. Procédé pour lutter contre la croissance de plantes nuisibles, avant ou après émergence, caractérisé en ce que le terrain infesté est traité avec une quantité efficace d'un composé herbicide ou d'une composition contenant un composé herbicide, choisi parmi ceux présentant la formule générale:

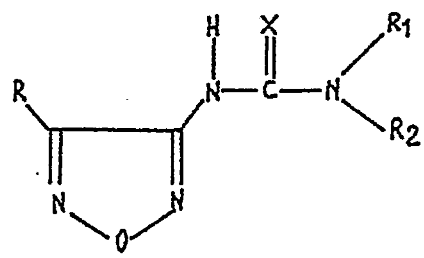

dans laquelle:

X est un atome d'oxygène ou de soufre;

R est un groupe alkyle linéaire ou ramifié comportant de 1 à 6 atomes de carbone;

le groupe phényle;
un groupe phényle comportant un ou plusieurs substituants qui peuvent être identiques ou différents les uns des autres, choisis parmi les atomes d'halogène et les groupes alkyle en $C_{1-4}$, alcoxy en $C_{1-4}$, halogénoalkyle en $C_{1-5}$ et nitro;
$R_1$ est un groupe alkyle linéaire ou ramifié comportant de 1 à 10 atomes de carbone;
un groupe cycloalkyle comportant de 3 à 8 atomes de carbone;
le groupe phényle;
le groupe benzyle;
le groupe naphthyle; ou
un groupe phényle, benzyle ou naphthyle, comportant un ou plusieurs substituants qui peuvent être identiques ou différents les uns des autres, choisis parmi les atomes d'halogène et les groupes alkyle en $C_{1-4}$, alcoxy en $C_{1-4}$, halogénoalkyle en $C_{1-4}$ et nitro;
$R_2$ est un atome d'hydrogène;
un groupe alkyle linéaire ou ramifié comportant de 1 à 6 atomes de carbone; ou
le groupe phényle; ou bien
$R_1$ et $R_2$, pris conjointement avec l'atome d'azote auquel ils sont liés, forment un noyau hétérocyclique à 8 chaînons.

7. Procédé conforme à la revendication 6, caractérisé en ce que le terrain est traité avec de 0,1 à 5 kg/ha du composé herbicide.